# EUROPÄISCHE PATENTSCHRIFT

(11) **EP 0 842 249 B1**
(45) Veröffentlichungstag und Bekanntmachung des Hinweises auf die Patenterteilung: **15.03.2000**
(21) Anmeldenummer: 96927016.4
(22) Anmeldetag: 16.07.1996
(51) Int. Cl.: C11D 1/835, C11D 1/65, A61K 7/50, B01F 17/00

(54) **FLIESSFÄHIGES PERLGLANZKONZENTRAT**
POURABLE NACREOUS LUSTRE CONCENTRATE
CONCENTRE COULANT A ASPECT NACRE

(30) Priorität: 25.07.1995 DE 19527120
(43) Veröffentlichungstag der Anmeldung: 20.05.1998
(73) Patentinhaber: Cognis Deutschland GmbH, 40589 Düsseldorf (DE)
(72) Erfinder: ANSMANN, Achim, D-40699 Erkrath (DE); KAWA, Rolf, D-40789 Monheim (DE); GONDEK, Helga, D-40589 Düsseldorf (DE)
(86) Internationale Anmeldenummer: EP9603117
(87) Internationale Veröffentlichungsnummer: WO9705224

(56) Entgegenhaltungen:
- EP-A- 0 221 465
- EP-A- 0 300 379
- EP-A- 0 332 805
- EP-A- 0 376 083
- WO-A-95/03782
- DE-A- 4 103 551
- DE-C- 4 400 632

## Beschreibung

Gegenstand der Erfindung ist ein Perlglanzkonzentrat in Form einer fließ- oder pumpfähigen, wäßrigen Dispersion mit einem Gehalt von 5 - 50 Gew.-% an perlglanzbildenden Komponenten.

Wäßrigen Zubereitungen von Tensiden und kosmetischen Präparaten kann man durch Einarbeitung von Substanzen, die nach dem Abkühlen in Form feiner, perlmuttartig aussehender Kristalle ausfallen und in den Zubereitungen dispergiert bleiben, ein perlglänzendes, ästhetisch ansprechendes Aussehen verleihen. Als perlglanzbildende Stoffe eignen sich z. B. die Mono-, Di- und gegebenenfalls Triester von Ethylenglykol, Propylenglykol und oligomere Alkylenglykole dieser Art oder Glycerin mit C₁₄-C₂₂-Fettsäuren, Fettsäuren sowie Monoalkanolamide von Fettsäuren.

Es ist auch bekannt, die genannten Perlglanzbildner in Wasser oder in wäßrigen Tensidlösungen stabil zu dispergieren und die auf diese Weise erhaltenen konzentrierten Perlglanzdispersionen den perlglänzend auszustattenden Zubereitungen ohne Erwärmung zuzusetzen, so daß sich das für die Einarbeitung sonst erforderliche Erwärmen und Abkühlen zur Bildung der Perlglanzkristalle erübrigt.
Die Europäische Patentschrift **EP 0332805 A2** beschreibt Perlglanzdispersionen, welche wäßrige Dispersionen aus einer perlglanzbildenden Komponente, einem Emulgator und einem Alkohol enthält. Die Schrift **WO 95/03782** offenbart ein Perlglanzkonzentrat aus Alkylpolysacchariden, perlglanzbildenden Komponenten und Alkyl(ether)sulfaten.
Auch wenn heute bereits eine größere Auswahl an Perlglanzkonzentraten zur Verfügung stehen, die in vielen Punkten die gestellten Anforderungen erfüllen, so werden doch weiterhin Versuche unternommen, Konzentrate zu entwickeln, die sich durch einen besonders brillanten Perlglanz auszeichnen.
Weiterhin ist es eine Bestrebung, Perlglanzkonzentrate mit hervorragenden Eigenschaften bereitzustellen, deren Emulgator-Basis vollständig oder überwiegend auf nichtionogenen Tensiden beruht. Solche Perlglanzkonzentrate können dann in der Regel ohne Rücksichtnahme auf die Emulgatortypen im Endprodukt eingesetzt werden.
Weiterhin ist es ein Bestreben, die Perlglanzkonzentrate auf einer Tensidbasis zu formulieren, die sich durch eine möglichst gute biologische Abbaubarkeit auszeichnet.

Es wurde nun gefunden, daß Perlglanzkonzentrate auf Basis von Fettsäure-N-alkylpolyhydroxyalkylamid-Emulgatoren diese Anforderungen in besonderem Maße erfüllen.

Gegenstand der Anmeldung sind somit Perlglanzkonzentrate in Form fließfähiger, wäßriger Dispersionen, enthaltend
(a) 5 bis 50 Gew.-% perlglanzbildende Komponenten,
(b) 5 bis 55 Gew.-% Fettsäure-N-alkylpolyhdroxyalkylamide der Formel **(I)** 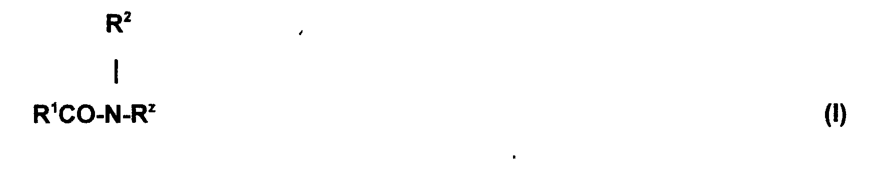 in der R¹CO für einen aliphatischen Acylrest mit 6 bis 22 Kohlenstoffatomen, R² für einen Alkyl- oder Hydroxyalkylrest mit 1 bis 4 Kohlenstoffatomen und R^{z} für einen linearen oder verzweigten Polyhydroxyalkylrest mit 3 bis 12 Kohlenstoffatomen und 3 bis 10 Hydroxylgruppen, und
(c) 0,1 bis 40 Gew.-% niedermolekulare, mehrwertige Alkohole,
   mit der Maßgabe, daß keine Alkylpolyglykoside als nichtionische Emulgatoren zugegen sind.

Unter perlglanzbildenden Komponenten werden schmelzbare Fett- oder Wachskörper verstanden, die beim Abkühlen ihrer wäßrigen Lösungen oder Emulsionen in einem Temperaturbereich von etwa 30 bis 90 °C in Form feiner, perlglänzender Körper auskristallisieren.

Zu diesen schmelzbaren Fett- oder Wachskörpem gehören:
- Ester der Formel **(II),**

   **R**^{**3**}**(OC**_{**n**}**H**_{**2n**}**)**_{**x**}**OR**^{**4**} **(II)**

   in der R³ für einen linearen Fettacylrest mit 14 bis 22 Kohlenstoffatomen, R⁴ für Wasserstoff oder R³, n für 2 oder 3 und x für eine Zahl von 1 bis 4 steht.
- Monoalkanolamide der Formel **(III),**

   **R**^{**5**}**CONHX** **(III)**

   in der R⁵ für eine Alkylgruppe mit 8 bis 22 Kohlenstoffatomen und X für eine Gruppe -CH₂CH₂OH, -CH₂CH₂CH₂OH oder -C(CH₃)₂OH steht.
- lineare, gesättigte Fettsäuren mit 14 bis 22 C-Atomen,
   sowie
- Mono-, Di- und Triester des Glycerins mit linearen, gesättigten Fettsäuren mit 14 bis 22 C-Atomen.

Als Ester der allgemeinen Formel R³(OCₙH₂ₙ)ₓOR⁴ können z. B. die Mono- und Diester des Ethylenglykols und Propylenglykols mit höheren Fettsäuren, z. B. mit Palmitinsäure, Stearinsäure oder Behensäure, oder die Diester des Diethylenglykols oder des Triethylenglykols mit solchen Fettsäuren eingesetzt werden. Geeignet sind auch Mischungen von Mono- und Diestern der genannten Glykole mit Fettsäuregemischen, z. B. mit gehärteter Talgfettsäure, Palmfettsäure oder mit der gesättigten C₁₄-C₁₈-Fettsäurefraktion der Talgfettsäure. Bevorzugt geeignet sind der Ethylenglykolmono- und/oder -diester der Palmitin- und/oder Stearinsäure.

Bevorzugte Monoalkanolamide sind die Monoethanolamide. Diese Verbindungen können einen einheitlichen Alkylrest enthalten. Es ist jedoch üblich, bei der Herstellung der Alkanolamide von Fettsäuregemischen aus natürlichen Quellen, z.B. Kokosfettsäuren, auszugehen, so daß entsprechende Mischungen bezüglich der Alkylreste vorliegen.

Als lineare Fettsäuren können z. B. Palmitinsäure, Stearinsäure, Arachinsäure oder Behensäure eingesetzt werden, geeignet sind aber auch technische Fettsäureschnitte, die ganz oder überwiegend aus Fettsäuren mit 16 bis 22 C-Atomen bestehen, z. B. Palmitin-Stearinsäure-Fraktionen, wie sie aus Talgfettsäure oder Palmfettsäure durch Abtrennung der bei +5 °C flüssigen Fettsäuren gewonnen werden oder Palmitin-Stearinsäure-Fraktionen, wie sie durch Härten von Talgfettsäure oder Palmfettsäure erhältlich sind.

Zu den in der erfindungsgemäßen Lehre verwendbaren Estern des Glycerins gehören die Mono-, Di- und insbesondere Triester mit Laurinsäure, Myristinsäure, Palmitinsäure, Stearinsäure und Behensäure, sowie mit Mischungen dieser Fettsäuren.

Die erfindungsgemäßen Perlglanzkonzentrate können sowohl ausschließlich Vertreter einer dieser Verbindungsklassen als auch Mischungen von Vertretern mehrerer dieser Verbindungsklassen enthalten.

Bevorzugte perlglanzbildende Komponenten sind Vertreter der drei erstgenannten Klassen.

Fettsäure-mono- oder -dialkanolamide und deren Derivate werden aber in jüngster Zeit verdächtigt, an der Bildung von Nitroaminen beteiligt zu sein. Es kann daher erwünscht sein, kosmetische Zubereitungen ohne solche Alkanolamine und Alkanolamin-Derivate zu formulieren. Aus diesem Grunde können Verbindungen der Klassen 1 und 3 besonders bevorzugte perlglanzbildende Komponenten sein.

Ganz besonders bevorzugt sind solche Perlglanzkonzentrate, deren perlglanzbildende Komponenten zu mindestens 70 Gew.-%, insbesondere zu mindestens 90 Gew.-%, aus Ethylenglykoldistearat bestehen.

Insbesondere können auch Mischungen von Ethylenglykolfettsäureestern bevorzugt sein, wie sie in der deutschen Patentanmeldung **DE 19511569** offenbart sind.

Als weitere zwingende Komponente enthalten die erfindungsgemäßen Perlglanzkonzentrate Emulgatoren vom Typ der Fettsäure-N-alkylpolyhydroxyalkylamide. Diese Verbindungen haben die allgemeine Formel **(I)** 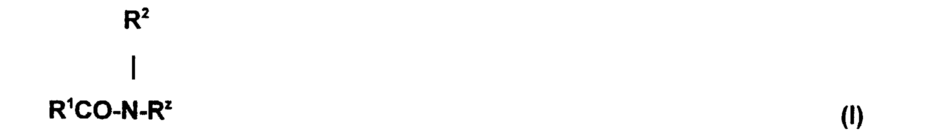 in der R¹CO für einen aliphatischen Acylrest mit 6 bis 22 Kohlenstoffatomen, R² für einen Alkyl- oder Hydroxyalkylrest mit 1 bis 4 Kohlenstoffatomen und R^{z} für einen linearen oder verzweigten Polyhydroxyalkylrest mit 3 bis 12 Kohlenstoffatomen und 3 bis 10 Hydroxylgruppen steht.

Diese Verbindungen sind literaturbekannt und können üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden.

Vorzugsweise leiten sich die Fettsäure-N-alkylpolyhydroxyalkylamide von reduzierenden Zuckern mit 5 bis 6 Kohlenstoffatomen, insbesondere von der Glucose, ab. Fettsäure-N-alkylglucamide der Formel **(IV)** 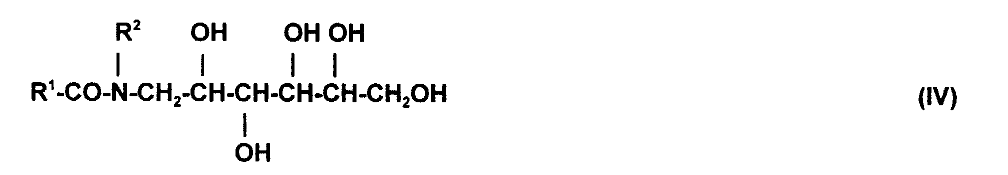 sind daher erfindungsgemäß bevorzugte Fettsäure-N-alkylpolyhydroxyalkylamide. Weiterhin sind solche Fettsäure-N-alkylpolyhydroxyalkylamide der Formel **(I)** bzw. Fettsäure-N-alkylglucamide der Formel **(IV)**, in denen R² für Wasser oder eine Alkylgruppe steht. Ebenfalls bevorzugt sind solche Verbindungen, bei denen die Gruppe R¹-CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure beziehungsweise derer Mischungen, wie sie insbesondere bei der technischen Aufarbeitung natürlicher Öle und Fette gewonnen werden, steht. Acylreste R¹-CO mit 12 bis 18 Kohlenstoffatomen sind besonders bevorzugt.

Ganz besonders bevorzugt sind Fettsäure-N-alkylglucamide, der Formel **(IV)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin haben sich erfindungsgemäß auch solche Verbindungen bewährt, bei denen der Polyhydroxyalkylrest sich von der Maltose bzw. Palatinose ableiten.

Weitere Informationen über Herstellung und Eigenschaften dieser Verbindungen können der Literatur entnommen werden. Auf die diesbezügliche Offenbarung der deutschen Patentschrift **DE 4400632 C1** wird ausdrücklich Bezug genommen.

Die erfindungsgemäßen Perlglanzkonzentrate können die genannten Fettsäure-N-alkylpolyhydroxyalkylamide als einzige Emulgatoren enthalten. Dies stellt eine bevorzugte Ausführungsform der Erfindung dar.

Je nach dem spezifischen Einsatzgebiet der Perlglanzkonzentrate können in einer weiteren bevorzugten Ausführungsform aber auch noch weitere Emulgatoren enthalten sein. Bei diesen Emulgatoren kann es sich prinzipiell sowohl um nichtionogene, als auch um ionische Emulgatoren handeln. Als ionische Emulgatoren können dabei sowohl kationische, als auch insbesondere anionische, zwitterionische und ampholytische Emulgatoren eingesetzt werden.

Erfindungsgemäß einzusetzende anionische Emulgatoren sind beispielsweise Alkylsulfate und Alkylpolyethylenglykolethersulfate mit 8 bis 22 C-Atomen in der Alkylkette und 1 bis 15, insbesondere 1 bis 6 Ethylenglykolethergruppen im Molekül, die in Form ihrer Alkali-, Magnesium-, Ammonium-, Mono-, Di- oder Trialkanolammoniumsalze mit 2 bis 3 C-Atomen in der Alkanolgruppe eingesetzt werden. Weitere geeignete Aniontenside sind Alkansulfonate, α-Olefinsulfonate, α-Sulfofettsäuremethylester, Fettalkohol(polyglykolether)carboxylate, Sulfobernsteinsäuremono- und -dialkylester, Sulfobernsteinsäureester-Salze, Acylisethionate, Acyltauride und Acylsarcoside, jeweils mit 8 bis 22, insbesondere 12 bis 18, C-Atomen in der Alkyl- bzw. Acylkette. Auch Seifen können als Emulgatoren verwendet werden. Dies kann z. B. dadurch erreicht werden, daß man einen kleinen Teil, d.h. etwa 1 bis 20 Gew.-%, der linearen, gesättigten Fettsäuren durch zugesetztes Alkalihydroxid verseift und auf diese Weise in einen anionischen Emulgator überführt.

Bevorzugte anionische Emulgatoren sind die Alkylpolyethylenglykolethersulfate wie beispielsweise das Natriumlaurylpolyglykolethersulfat.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyl-dimethylammonium-glycinat, N-Acylaminopropyl-N,N-dimethylammoniumglycinate, beispielsweise das Kokosacylaminopropyl-dimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls geeignete Emulgatoren sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Als kationische Emulgatoren sind quartäre Ammoniumtenside, beispielsweise Alkyltrimethylammoniumchloride und Dialkyldimethylammoniumchloride, z. B. Cetyltrimethylammoniumchlorid, Stearyltrimethylammoniumchlorid, Distearyldimethylammoniumchlorid, Lauryldimethylammoniumchlorid und Lauryldimethylbenzylammoniumchlorid, Cetylpyridiniumchlorid und Talgalkyltris-(oligooxyalkyl)-ammoniumphosphat zu nennen. Weiterhin können die sehr gut biologisch abbaubaren quarternären Esterverbindungen, wie beispielsweise die unter dem Warenzeichen Stepantex® vertriebenen Dialkylammoniummethosulfate und Methyl-hydroxyalkyldialkoyloxyalkyl-ammoniummethosulfate als kationische Tenside eingesetzt werden.

Schließlich können auch nichtionogene Emulgatoren verwendet werden, die als hydrophile Gruppe z. B. eine Polyolgruppe, eine Polyalkylenglykolethergruppe oder eine Kombination aus Polyol- und Polyglykolethergruppe enthalten. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- Glycerinmono- und -diester und Sorbitanmono- und -diester von gesättigten und ungesättigten C₈-C₂₂-Fettsäuren und deren Ethylenoxidanlagerungsprodukte sowie
- Anlagerungsprodukte von 5 bis 60 Mol Ethylenoxid an Ricinusöl und gehärtetes Ricinusöl.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen und tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Im Rahmen der erfindungsgemäßen Lehre können die Perlglanzkonzentrate Vertreter einer oder mehrerer der genannten Tensidklassen enthalten.

Da Perlglanzkonzentrate überwiegend zu Formulierungen mit anionischen Tensiden hinzugegeben werden, enthalten die erfindungsgemäßen Perlglanzkonzentrate in einer bevorzugten Ausführungsform lediglich anionische Emulgatoren als weitere Emulgatoren.

Weiterhin haben sich die erfindungsgemäßen Perlglanzkonzentrate, die lediglich nichtionogene, zwitterionische und/oder ampholytische Tenside enthalten, als besonders universell einsetzbar und mit wäßrigen Zubereitungen wasserlöslicher oberflächenaktiver Stoffe beliebiger Art und lonogenität als besonders gut verträglich erwiesen. Gemäß einer weiteren bevorzugten Ausführungsform enthalten die erfindungsgemäßen Perlglanzkonzentrate lediglich diese Arten von Emulgatoren, insbesondere nur nichtionogene Emulgatoren als weitere Emulgatoren.

Weiterhin können die erfindungsgemäßen Perlglanzkonzentrate in einer bevorzugten Ausführungsform zusätzlich niedermolekulare, mehrwertige Alkohole enthalten. Dadurch kann insbesondere das Pump- und Fließverhalten der Perlglanzkonzentrate sowie ihre Haltbarkeit verbessert werden.

Niedermolekulare, mehrwertige Alkohole einer bevorzugt eingesetzten Gruppen enthalten 2-12 Kohlenstoffatome und 2-10 Hydroxylgruppen. Solche Alkohole sind beispielsweise Ethylenglykol, 1,2- und 1,3-Propylenglykol, Glycerin, Erythrit, Arabit, Adonit, Xylit, Sorbit, Mannit, Dulcit, Glucose und Saccharose. Die Verwendung von Glycerin, 1,2-Propylenglykol, 1,3-Propylenglykol, Sorbit und/oder Glucose ist besonders bevorzugt.

Die Verwendung von Glycerin als niedermolekularer, mehrwertiger Alkohol führt zu Perlglanzkonzentraten, die den Endprodukten einen besonders brillanten Perlglanz verleihen.

Eine weitere Gruppe bevorzugt eingesetzter niedermolekularer, mehrwertiger Alkohole wird von oligomeren Ethern, insbesondere auf Basis von Ethylenglykol, Propylenglykol und Glycerin, gebildet. Dabei sind vor allem solche Produkte geeignet, deren mittlere Molmasse unterhalb von etwa 700 Dalton liegt. Vor allem die Oligomeren, insbesondere die Di-, Tri- und Tetrameren, von Ethylenglykol und Glycerin sind erfindungsgemäß verwendbar.
Die niedermolekularen, mehrwertigen Alkohole werden erfindungsgemäß in Mengen von 0,1 bis 40 Gew.-%, bezogen auf die gesamte Zubereitung, zugesetzt. Mengen von 0,1 bis 10 Gew.-% an niedemolekularen, mehrwertigen Alkoholen wirken sich insbesondere positive auf das Pump- und
Fließverhalten aus, während Mengen von mehr als 10 Gew.-%, jeweils bezogen auf das gesamte Perlglanzkonzentrat, in vielen Fällen eine spezielle Konservierung ersetzen können.

Daneben enthalten die erfindungsgemäßen Perlglanzkonzentrate im wesentlichen Wasser.

Weiterhin können in untergeordneten Mengen Puffersubstanzen zur Einstellung des pH-Wertes auf Werte zwischen 2 und 8, z. B. Citronensäure und/oder Natriumcitrat, sowie anorganische Salze, beispielsweise Natriumchlorid, als Verdikkungsmittel enthalten sein.

In einer ersten, bevorzugten Ausführungsform sind die erfindungsgemäßen Perlglanzkonzentrate mit den üblichen, dem Fachmann bekannten Konservierungsmitteln versehen. Solche Konservierungsmittel sind beispielsweise Ameisensäure, Benzoesäure und pHB-Ester.

In einer zweiten bevorzugten Ausführungsform sind die Perlglanzkonzentrate konservierungsmittelfrei. Unter konservierungsmittelfrei werden in diesem Zusammenhang Perlglanzkonzentrate verstanden, denen keine Konservierungsmittel zugesetzt wurden. Sie enthalten daher bevorzugt keine Konservierungsmittel bzw. Konservierungsmittel nur in solchen Mengen, wie sie aufgrund der gewählten einzelnen Rohstoffe mit diesen eingebracht werden.

Die erfindungsgemäßen Perlglanzkonzentrate eignen sich zur Herstellung getrübter und perlglänzender flüssiger, wäßriger Zubereitungen wasserlöslicher oberflächenaktiver Stoffe. Sie können z.B. in flüssige Wasch- und Reinigungsmittel wie Spülmittel, flüssige Feinwaschmittel und flüssige Seifen, bevorzugt aber in flüssige Körperreinigungs- und Pflegemittel wie z.B. Haarwaschmittel (Shampoos), flüssige Hand- und Körperwaschmittel, Duschbadzubereitungen, Badezusätze (Schaumbäder), Haarspülmittel oder Haarfärbezubereitungen eingearbeitet werden.

Zur Erzeugung von Perlglanz werden den klaren wäßrigen Zubereitungen bei 0 bis 40 °C die erfindungsgemäßen Perlglanzkonzentrate in einer Menge von 0,5 bis 20 Gew.-%, insbesondere von 1,5 bis 10 Gew.-%, der Zubereitung zugesetzt und unter Rühren darin verteilt. Je nach Zubereitung und Einsatzkonzentration entsteht ein metallisch glänzender, dichter bis schwach glänzender, extrem dichter Perlglanz.

Die folgenden Beispiele sollen den erfindungsgemäßen Gegenstand erläutern.

### Beispiele

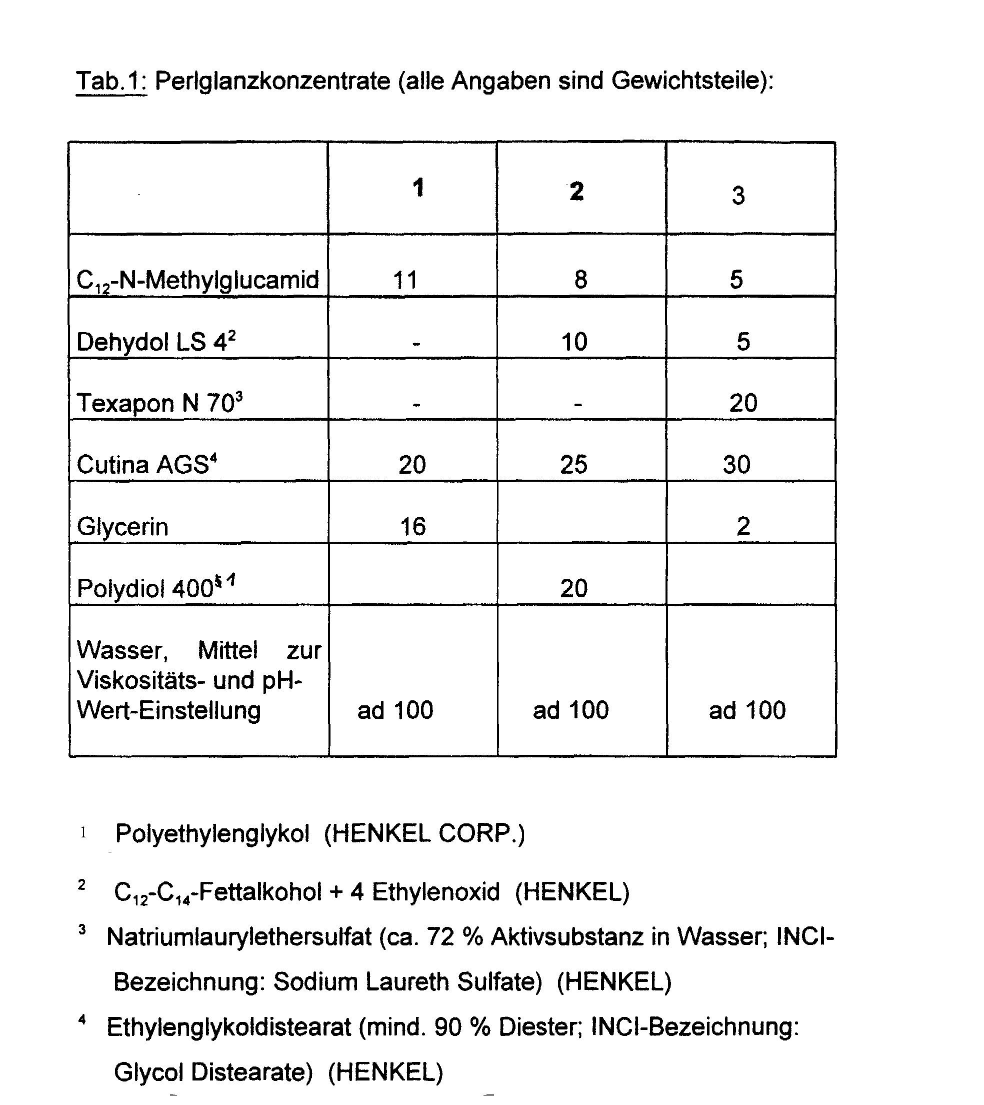

## Patentansprüche

1. Perlglanzkonzentrate in Form fließfähiger, wäßriger Dispersionen, enthaltend
(a) 5 bis 50 Gew.-% perlglanzbildende Komponenten,
(b) 5 bis 55 Gew.-% Fettsäure-N-alkylpolyhdroxyalkylamide der Formel **(I)** 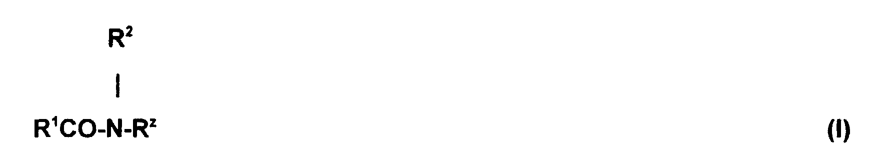 in der R¹CO für einen aliphatischen Acylrest mit 6 bis 22 Kohlenstoffatomen, R² für einen Alkyl- oder Hydroxyalkylrest mit 1 bis 4 Kohlenstoffatomen und R^{z} für einen linearen oder verzweigten Polyhydroxyalkylrest mit 3 bis 12 Kohlenstoffatomen und 3 bis 10 Hydroxylgruppen, und
(c) 0,1 bis 40 Gew.-% niedermolekulare, mehrwertige Alkohole,
mit der Maßgabe, daß keine Alkylpolyglykoside als nichtionische Emulgatoren zugegen sind.

2. Perlglanzkonzentrate nach Anspruch 1, **dadurch gekennzeichnet,** daß sie als perlglanzbildende Komponenten Ester der Formel **(II)** enthalten,
**R**^{**3**}**(OC**_{**n**}**H**_{**2n**}**)**_{**x**}**OR**^{**4**} **(II)**
in der R³ für einen linearen Fettacylrest mit 14 bis 22 Kohlenstoffatomen, R⁴ für Wasserstoff oder R³, n für 2 oder 3 und x für eine Zahl von 1 bis 4 steht.

3. Perlglanzkonzentrate nach den Ansprüchen 1 und 2, **dadurch gekennzeichnet,** daß sie als perlglanzbildende Komponenten Monoalkanolamide der Formel (III) enthalten,
**R**^{**6**}**CONHX** **(III)**
in der R⁵ für eine Alkylgruppe mit 8 bis 22 Kohlenstoffatomen und X für eine Gruppe -CH₂CH₂OH, -CH₂CH₂CH₂OH oder -C(CH₃)₂OH steht.

4. Perlglanzkonzentrate nach den Ansprüchen 1 bis 3, **dadurch gekennzeichnet,** daß sie als perl-glanzbildende Komponenten lineare, gesättigte Fettsäuren mit 12 bis 14 Kohlenstoffatomen enthalten.

5. Perlglanzkonzentrate nach den Ansprüchen 1 bis 4, **dadurch gekennzeichnet,** daß sie als perlglanzbildende Komponenten C₁₂₋₂₂ Fettsäuremono-, -di- oder -triglyceride enthalten.

6. Perlglanzkonzentrate nach den Ansprüchen 1 bis 5, **dadurch gekennzeichnet,** daß sie als niedermolekulare Alkohole Glycerin, 1,2-Propylenglycol, 1,3-Propylenglycol, Glucose oder Sorbit enthalten.

## Claims

1. Pearlescer concentrates in the form of free-flowing aqueous dispersions containing
(a) 5 to 50% by weight of pearlescing components,
(b) 5 to 55% by weight of fatty acid-N-alkyl polyhydroxyalkylamides corresponding to formula (I): 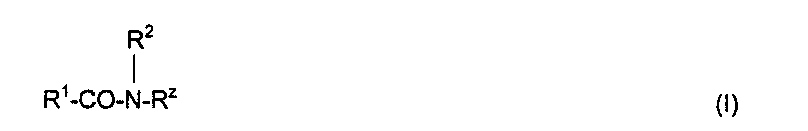 in which R¹-CO is an aliphatic acyl group containing 6 to 22 carbon atoms, R² is an alkyl or hydroxyalkyl group containing 1 to 4 carbon atoms and R^{z} is a linear or branched polyhydroxyalkyl radical containing 3 to 12 carbon atoms and 3 to 10 hydroxyl groups, and
(c) 0.1 to 40% by weight of low molecular weight polyhydric alcohols, with the proviso that no other nonionic emulsifiers are present.

2. Pearlescer concentrates as claimed in claim 1, characterized in that they contain esters corresponding to formula (II):
R³-(OCₙH₂ₙ)ₓOR⁴ (II)
in which R³ is a linear fatty acyl group containing 14 to 22 carbon atoms, R⁴ is hydrogen or has the same meaning as R³, n is the number 2 or 3 and x is a number of 1 to 4,
as pearlescing components.

3. Pearlescer concentrates as claimed in claims 1 and 2, characterized in that they contain monoalkanolamides corresponding to formula (III):
R⁵-CO-NH-X (III)
in which R⁵ is an alkyl group containing 8 to 22 carbon atoms and, more particularly, 8 to 18 carbon atoms and X is a -CH₂-CH₂-OH group, a -CH₂- CH₂-CH₂-OH group or a -C(CH₃)₂-OH group,
as pearlescing components.

4. Pearlescer concentrates as claimed in claims 1 to 3, characterized in that they contain linear saturated fatty acids containing 12 to 14 carbon atoms as pearlescing components.

5. Pearlescer concentrates as claimed in claims 1 to 4, characterized in that they contain C₁₂₋₂₂ fatty acid mono-, di- or triglycerides as pearlescing components.

6. Pearlescer concentrates as claimed in claims 1 to 5, characterized in that they contain glycerol, 1,2-propylene glycol, 1,3-propylene glycol, glucose or sorbitol as low molecular weight alcohols.

## Revendications

1. Concentrés conférant un effet nacré sous forme de dispersions aqueuses fluides contenant
(a) de 5 à 50 % en poids de composants conférant un aspect nacré,
(b) de 5 à 55 % en poids de N-alkylpolyhydroxyalkylamides d'acides gras de formule (I) 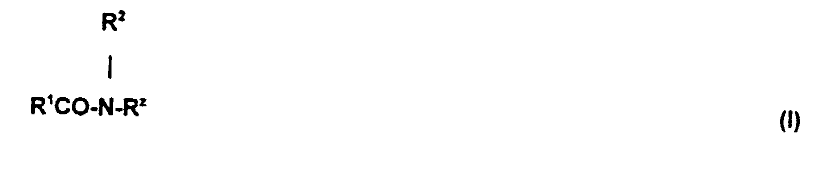 dans laquelle R¹CO représente un radical acyle aliphatique ayant de 6 à 22 atomes de carbone, R² représente un radical alkyle ou hydroxyalkyle ayant de 1 à 4 atomes de carbone et R^{z} représente un radical polyhydroxyalkyle linéaire ou ramifié ayant de 3 à 12 atomes de carbone et de 3 à 10 groupes hydroxyles, et
(c) de 0,1 à 40 % en poids d'alcools polyvalents à faible poids moléculaire,
sous réserve qu'aucun alkyle polyglycoside ne soit présent comme émulsifiant non ionique.

2. Concentrés donnant un effet nacré selon la revendication 1,
caractérisé en ce qu'
ils contiennent comme composants conférant un effet nacré des esters de formule (II),
R³(OCₙH₂ₙ)ₓOR⁴ (II)
dans laquelle R³ représente un radical acyle gras linéaire ayant de 14 à 22 atomes de carbone, R⁴ représente un hydrogène ou R³, n vaut 2 ou 3 et x représente un nombre de 1 à 4.

3. Concentrés donnant un effet nacré selon les revendications 1 et 2,
caractérisé en ce qu'
ils contiennent comme composant donnant un effet nacré des monoalcanolamides de formule (III),
R⁵CONHX (III)
dans laquelle R⁵ représente un groupe alkyle ayant de 8 à 22 atomes de carbone et X représente un groupe -CH₂CH₂OH, CH₂CH₂CH₂OH ou -C(CH₃)₂OH.

4. Concentrés donnant un effet nacré selon la revendication 3,
caractérisés en ce qu'
ils contiennent comme composant conférant un effet nacré des acides gras saturés linéaires ayant de 12 à 14 atomes de carbone.

5. Concentrés conférant un effet nacré selon les revendications 1 à 4,
caractérisés en ce qu'
ils contiennent comme composant conférant un effet nacré des mono-, di- ou triglycérides d'acides gras en C₁₂ à C₂₂,

6. Concentrés conférant un effet nacré selon les revendications 1 à 5,
caractérisés en ce qu'
ils contiennent comme alcools de faible poids moléculaire la glycérine, le 1,2-propylène glycol, le 1,3-propylène glycol, le glucose ou le sorbitol.
